# EUROPÄISCHE PATENTANMELDUNG

(11) **EP 4 445 849 A1**
(43) Veröffentlichungstag der Anmeldung: **16.10.2024**
(21) Anmeldenummer: 23168050.5
(22) Anmeldetag: 14.04.2023
(51) Int. Cl.: A61B 17/122

(54) **MEDIZINISCHER CLIP**

(71) Anmelder: Aesculap AG, 78532 Tuttlingen (DE)
(72) Erfinder: Scholten, Thomas, 78532 Tuttlingen (DE); Beger, Jens, 78532 Tuttlingen (DE); Happle, Alexander, 78183 Hüfingen (DE); Buening, Teresa, 78532 Tuttlingen (DE); Dalmann, Natalia, 78532 Tuttlingen (DE)
(74) Vertreter: Hoeger, Stellrecht & Partner Patentanwälte mbB

(57) **Zusammenfassung**

Die Erfindung betrifft einen medizinischen Clip, insbesondere in Form eines Aneurysmenclips, welcher einen ersten Klemmarm, einen zweiten Klemmarm und ein vorspannendes Element mit einem ersten und einem zweiten Ende umfasst, wobei der erste Klemmarm ein erstes Klemmarmende aufweist, welches über einen ersten Verbindungsabschnitt mit dem ersten Ende des vorspannenden Elements verbunden ist, wobei der zweite Klemmarm ein zweites Klemmarmende aufweist, welches über einen zweiten Verbindungsabschnitt mit dem zweiten Ende des vorspannenden Elements verbunden ist, wobei der Clip einen die zusammenwirkenden ersten und zweiten Verbindungsabschnitte umfassenden Durchsteckschluss umfasst mit mindestens einer ersten, am ersten Verbindungsabschnitt angeordneten oder ausgebildeten und von zwei ersten Schlussstegen seitlich begrenzten Schlussdurchbrechung und mindestens einem vom zweiten Verbindungsabschnitt umfassten, die erste Schlussdurchbrechung durchsetzenden zweiten Schlusssteg, wobei die erste Schlussdurchbrechung zwei aufeinander zu weisende weibliche Lagerflächen umfasst und wobei der mindestens eine zweite Schlusssteg zwei voneinander weg und auf die weiblichen Lagerflächen hin weisende männliche Lagerflächen umfasst.

## Beschreibung

Die vorliegende Erfindung betrifft einen medizinischen Clip, insbesondere in Form eines Aneurysmenclips, welcher einen ersten Klemmarm, einen zweiten Klemmarm und ein vorspannendes Element mit einem ersten und einem zweiten Ende umfasst, wobei der erste Klemmarm ein erstes Klemmarmende aufweist, welches über einen ersten Verbindungsabschnitt mit dem ersten Ende des vorspannenden Elements verbunden ist, wobei der zweite Klemmarm ein zweites Klemmarmende aufweist, welches über einen zweiten Verbindungsabschnitt mit dem zweiten Ende des vorspannenden Elements verbunden ist, wobei der Clip einen die zusammenwirkenden ersten und zweiten Verbindungsabschnitte umfassenden Durchsteckschluss umfasst mit mindestens einer ersten, am ersten Verbindungsabschnitt angeordneten oder ausgebildeten und von zwei ersten Schlussstegen seitlich begrenzten Schlussdurchbrechung und mindestens einen vom zweiten Verbindungsabschnitt umfassten, die erste Schlussdurchbrechung durchsetzenden zweiten Schlusssteg, wobei die erste Schlussdurchbrechung zwei aufeinander zu weisende weibliche Lagerflächen umfasst und wobei der mindestens eine zweite Schlusssteg zwei voneinander weg und auf die weiblichen Lagerflächen hin weisende männliche Lagerflächen umfasst, wobei mindestens zwei Lagerflächenpaare mit jeweils einer weiblichen und einer männlichen Lagerfläche gebildet sind und wobei jeweils die zusammenwirkenden weiblichen und männlichen Lagerflächen parallel zueinander verlaufen und eine Lagerebene definieren.

Ein medizinischer Clip der eingangs beschriebenen Art ist beispielsweise aus der WO 2022/096357 A1 bekannt. Ein weiterer medizinischer Clip mit einem Durchsteckschluss ist in der DE 20 2004 015 274 U1 beschrieben. Bei diesem Clip übernimmt das vorspannende Element zusätzlich noch die Funktion eines Lagerstifts für die beiden zusammenwirkenden und um eine Längsachse des vorspannenden Elements verdrehbaren Gelenkringe, an denen jeweils ein Klemmarm angeordnet oder ausgebildet ist. Der Gelenkring 23 bildet dabei zusammen mit dem Führungsplättchen die Schlussdurchbrechung, der Gelenkring 22 den die Schlussdurchbrechung durchsetzenden zweiten Verbindungsabschnitt. Ferner sind dem aus der DE 20 2004 015 274 U1 sehr ähnliche medizinische Clips mit einem Durchsteckschluss in der DE 199 35 418 A1 in den Figuren 1 und 2 dargestellt und detailliert beschrieben.

Im einem Schlussbereich von Aneurysmenclips, insbesondere wenn die Schlussbereiche in Form von Durchsteckschlüssen ausgebildet sind, bewegen sich beim Öffnen und Schließen der Clips die Schlusskomponenten, also die Schlussstege am ersten Verbindungsabschnitt und der mindestens eine zweite Schlusssteg am zweiten Verbindungsabschnitt, aneinander vorbei. Insbesondere gleiten sie aneinander ab. Bei dieser Bewegung tritt Reibung auf. Mit anderen Worten streifen beim Öffnen und Schließen der Clips die Schlussstege mit ihren männlichen und weiblichen, zusammenwirkenden Lagerflächen übereinander, was zu Reibung, aber auch zu Abrieb und zu einem Kaltverschwei-ßen, dem sogenannten "Fressen" führt. Durch diese Effekte ergibt sich eine erhöhte Wiederholungenauigkeit bei der Messung der Schließkraft der Clips. Oder mit anderen Worten eine verringerte Wiederholgenauigkeit bei der Messung der Schließkraft.

Für derartige Clips ist es von Bedeutung, dass die vom vorspannenden Element ausgeübte vorspannende Kraft, insbesondere also eine Federkraft, einen vordefinierten Wert aufweist. Zur Ermittlung der Federkraft von Aneurysmenclips werden diese gemäß einer bestehenden Norm auf einen bestimmten Wert geöffnet und auf eine Prüfvorrichtung aufgesetzt. Die dabei ermittelte Kraft, nämlich die oben angegebene Schließkraft, muss dann innerhalb eines bestimmten, von der Norm vorgegebenen Toleranzfensters liegen. Andernfalls muss der Clip überarbeitet oder als unbrauchbar aussortiert werden.

Die Schließkraft der Clips muss wie angegeben innerhalb normativer Grenzen eingehalten werden. So darf eine maximale Abweichung nicht mehr als +/- 7,5% von einem Nennwert betragen. Außerdem darf die Schließkraft auch nach mehrmaligem Öffnen nur um einen geringen Betrag abfallen. Ein so vorgegebener Schließkraftverlust muss weniger als 5% betragen. Das Messprinzip zur Ermittlung der Schließkraft und die vorgegebenen Grenzen beziehungsweise Abweichungen sind in der Norm EN ISO 9713:2022 beschrieben.

Durch die dem Grunde nach unvermeidliche Reibung und die damit verbundene Wiederholungenauigkeit bei der Herstellung von Clips geht ein wichtiger Teil der verfügbaren Toleranz bei der Herstellung verloren. Diese Toleranz wird jedoch als Prozessfenster in der Produktion dringend benötigt.

Es ist bekannt, dass die Reibung umso größer ist, je stärker die zusammenwirkenden männlichen und weiblichen Lagerflächen eines Lagerflächenpaars gegeneinander gedrückt werden. Dieser Gegendruck kann jedoch nicht beliebig reduziert werden, weil die zusammenwirkenden Klemmarme, auch als Maulteile bezeichnet, dann relativ zueinander ein undefiniertes Spiel erhalten.

Es ist daher eine Aufgabe der vorliegenden Erfindung, einen medizinischen Clips der eingangs beschriebenen Art so zu verbessern, dass eine Wiederholungenauigkeit bei der Messung der Schließkraft verringert wird.

Diese Aufgabe wird bei einem medizinischen Clip der eingangs beschriebenen Art erfindungsgemäß dadurch gelöst, dass sich eine durch senkrechte Projektion der ersten Schlussstege in die Lagerebene definierte erste Schlussstegprojektionsfläche und sich eine durch senkrechte Projektion des mindestens einen zweiten Schlussstegs in die Lagerebene definierte zweite Schlussstegprojektionsfläche überlappen und eine Projektionsüberlappungsfläche definieren, dass mindestens eine der beiden zusammenwirkenden Lagerflächen mindestens eines, insbesondere jedes, der mindestens zwei Lagerflächenpaare mindestens eine Vertiefung aufweist derart, dass eine tatsächliche Größe der Lagerfläche parallel zur Lagerebene, welche Lagerfläche von dem die mindestens eine Vertiefung umfassenden Schlusssteg definiert ist, kleiner ist als die vom Schlusssteg, welcher die mindestens eine Vertiefung umfasst, in die Lagerebene projizierte Schlussstegprojektionsfläche, dass die beiden zusammenwirkenden und flächig aneinander anlegbaren oder anliegenden, zusammenwirkenden Lagerflächen mindestens eines, insbesondere jedes, Lagerflächenpaars eine Kontaktüberlappungsfläche definieren und dass ein Verhältnis der Kontaktüberlappungsfläche und der Projektionsüberlappungsfläche in einem Bereich von etwa 1/25 bis etwa 1/3 liegt.

Die vorgeschlagene Weiterbildung ermöglicht insbesondere eine Reduktion der auftretenden Reibung zwischen zusammenwirkenden Lagerflächen der mindestens zwei Lagerflächenpaare. Die plan aneinander abgleitenden Lagerflächen, die gegenüber den Abmessungen der jeweiligen Schlussstege verringert sind, führen zu einer Reduzierung der Haftreibung und erleichtern den Übergang zur Gleitreibung. Zum Messen der Schließkraft kann der Clip beispielsweise etwas geöffnet werden, sodass die Klemmarme auf zwei Haltestifte aufgesetzt werden können. Zur Messung der Schließkraft wird der Clip wieder geschlossen, wobei der Gleitvorgang im Schlussbereich in einen statischen Messzustand mit Haftreibung übergeht. Je geringer der Reibungskoeffizient beim Übergang von Gleitreibung zu Haftreibung ist, desto geringer ist das Reibmoment bei der Schließkraftmessung und damit auch der Einfluss auf die Messung der Schließkraft und somit die Streuung der Messergebnisse. Durch die Verringerung der Kontaktüberlappungsfläche im Vergleich zur Projektionsüberlappungsfläche im angegebenen Bereich lässt sich die Reibung minimieren und somit eine Wiederholungenauigkeit wie gewünscht verringern. Um die Verringerung der Kontaktüberlappungsfläche im Vergleich zur Projektionsüberlappungsfläche zu erreichen, muss entweder eine der beiden zusammenwirkenden Lagerflächen bezogen auf die zugeordnete Schlussstegprojektionsfläche auf einen Wert in einem Bereich von etwa 1/25 bis 1/3 verringert werden. Alternativ können auch die beiden zusammenwirkenden Lagerflächen jeweils in ihrer Größe auf etwa 1/5 bis etwa 0,58 verringert werden. Diese Verringerung kann durch die mindestens eine Vertiefung erreicht werden, die in einer der beiden Lagerflächen oder gegebenenfalls auch in beiden Lagerflächen vorgesehen wird. Vorzugsweise liegt das Verhältnis der Kontaktüberlappungsfläche und der Projektionsüberlappungsfläche in einem Bereich von etwa 10% bis etwa 20%. In den angegebenen Verhältnisbereichen ist noch eine hinreichend gute Führung durch Anlage der Lagerflächen aneinander bei jedoch wie gewünscht verminderter Reibung erreichbar. Insbesondere kann das Verhältnis der Kontaktüberlappungsfläche und der Projektionsüberlappungsfläche in einem Bereich von etwa 1/25 bis etwa 1/4 liegen, um die unerwünschten Reibungseffekte noch weiter zu reduzieren.

Auf einfache Weise lässt sich die Reibung vermindern, wenn die mindestens eine Vertiefung in Form einer Nut in der Lagerfläche oder in Form einer Abschrägung an der Lagerfläche ausgebildet ist. Insbesondere kann die Lagerfläche längs von zwei parallel oder im Wesentlichen parallel verlaufenden Seitenkanten, die sich insbesondere parallel zu einer Erstreckung des jeweiligen Verbindungsabschnitts vom vorspannenden Element zum jeweiligen Klemmarm erstrecken, ausgebildet sein. Mithin können also eine oder zwei Abschrägungen am jeweiligen Schlusssteg vorgesehen sein, um eine tatsächliche Größe der Lagerfläche im Zusammenwirken mit der ihr zugeordneten und ein Lagerflächenpaar bildenden Lagerfläche zu verringern. Die Abschrägung kann insbesondere eine ebene Fläche definieren oder eine gekrümmte Fläche definieren, welche vom jeweiligen Verbindungsabschnitt weg weisend konvex oder konkav gekrümmt sein kann.

Vorzugsweise erstreckt sich die Nut oder die Abschrägung parallel oder im Wesentlichen parallel zu einer vom jeweiligen Schlusssteg definierten Längsrichtung. Beispielsweise kann die Nut Längskanten der Lagerfläche, wenn sich diese S-förmig geschwungen vom vorspannenden Element zu den Klemmarmen erstreckt, zu beiden Seiten durchbrechen.

Günstig ist es, wenn die Abschrägung eine Schrägfläche definiert, welche mit der Lagerebene einen stumpfen Schrägenwinkel einschließt. Die Schrägfläche kann insbesondere eben oder gekrümmt verlaufen. Der Schrägenwinkel kann insbesondere einen Wert in einem Bereich von etwa 140° bis etwa 175° aufweisen. Wie vorgeschlagen ergibt sich so beispielsweise im Übergang der Lagerfläche zur Abschrägung ein nahezu gestreckter Winkel, wodurch Grate vermieden werden. Günstig ist es, wenn der Schrägenwinkel in einem Bereich von 165° bis etwa 172° liegt.

Ferner kann es vorteilhaft sein, wenn sich die Nut quer insbesondere senkrecht, zu einer vom jeweiligen Schlusssteg definierten Längsrichtung erstreckt. Insbesondere können so mehrere Nuten vorgesehen werden. Diese können sich insbesondere parallel zueinander erstrecken. Ferner können die Nuten im Querschnitt eine Kreissegmentform aufweisen, sodass sich im Übergang zur verbleibenden Lagerfläche insbesondere ein stumpfer, vorzugsweise gestreckter Winkel ergibt. So lassen sich Grate bei der Herstellung des Clips vermeiden.

Ferner ist es günstig, wenn die mindestens eine Vertiefung in Form eines Hohlkugelausschnitts ausgebildet ist. Insbesondere kann der Hohlkugelausschnitt, auch als Hohlkugelsegment bezeichnet, die Form einer halben Hohlkugel aufweisen oder einen Teil einer halben Hohlkugel, sodass eine hinterschnittfreie Vertiefung ausgebildet wird. Derartige Vertiefungen lassen sich auf einfache Weise ausbilden, beispielsweise mit einem Kugelfräser. Vorzugsweise ist eine Tiefe des Hohlkugelausschnitts wesentlich kleiner als ein Radius desselben. Insbesondere ist die Tiefe kleiner als 1/5 des Radius.

Ferner ist es günstig, wenn die mindestens eine Vertiefung durch eine den Schlusssteg durchsetzende Schlussstegdurchbrechung ausgebildet ist. Beispielsweise kann eine solche Schlussstegdurchbrechung durch eine den Schlusssteg durchsetzende Bohrung ausgebildet werden.

Auf einfache Weise lässt sich die Schlussstegdurchbrechung ausbilden, wenn sie einen kreisförmigen Querschnitt aufweist. So kann sie insbesondere durch eine Bohrung ausgebildet werden.

Um die tatsächlich wirksame Lagerfläche im Vergleich zur jeweiligen Schlussstegprojektionsfläche in gewünschter Weise zu reduzieren, ist es vorteilhaft, wenn mindestens eine der beiden zusammenwirkenden Lagerflächen mindestens eines, insbesondere jedes, der mindestens zwei Lagerflächenpaare eine Mehrzahl von Vertiefungen aufweist. Beispielsweise kann so die Größe der tatsächlich zur Reibung des jeweiligen Lagerflächenpaars beitragende Lagerfläche wie gewünscht reduziert werden, eine Gefahr eines Verhakens der zusammenwirkenden Lagerflächen jedoch minimiert werden.

Auf einfach Weise lässt sich der medizinische Clip ausbilden, wenn die Mehrzahl von Vertiefungen identisch ausgebildet ist.

Um eine Optimierung der Verringerung der Lagerfläche zu ermöglichen ist es günstig, wenn sich mindestens ein Teil der Mehrzahl von Vertiefungen unterscheidet. Eine Unterscheidung ist insbesondere hinsichtlich der Form und/oder Größe der Vertiefungen denkbar. So können insbesondere sowohl hohlkugelige Vertiefungen als auch Nuten an derselben Lagerfläche vorgesehen sein. Beispielsweise können mehr als zwei Nuten unterschiedlicher Form und Größe vorgesehen sein und auch mehr als zwei Vertiefungen, die in Form eines Hohlkugelausschnitts ausgebildet sind.

Um eine möglichst geringe Kontaktüberlappungsfläche zu erreichen, ist es vorteilhaft, wenn beide zusammenwirkenden Lagerflächen jedes Lagerflächenpaars mindestens eine Vertiefung umfassen.

Gemäß einer weiteren bevorzugten Ausführungsform der Erfindung kann vorgesehen sein, dass eine Breite der Schlussstege ohne Berücksichtigung der mindestens einen Vertiefung quer zu einer vom jeweiligen Schlusssteg definierten Längsrichtung in einem Bereich von etwa 0,9 mm bis etwa 2,1 mm liegt.

Ferner ist es vorteilhaft, wenn der der erste Klemmarm und der zweite Klemmarm in einer Grundstellung des Clips einander maximal angenähert sind, insbesondere aneinander anliegend, und entgegen der Wirkung des vorspannenden Elements von der Grundstellung in eine Öffnungsstellung voneinander weg bewegbar sind. Das vorspannende Element übt dann eine Kraft auf die beiden Klemmarme aus, um diese wieder automatisch von der Öffnungsstellung wieder in die Grundstellung zu überführen, wenn die Klemmarme nach dem Öffnen von einem Anwender wieder freigegeben werden. Genau diese Kraft ist dann die Schließkraft, die für den Clip mit möglichst hoher Wiederholgenauigkeit oder mit anderen Worten mit möglichst geringer Wiederholungenauigkeit - sowohl vorgegeben als auch gemessen werden soll.

Eine optimale Funktion des medizinischen Clips lässt sich insbesondere erreichen, wenn die Lagerebene eben oder im Wesentlichen eben ausgebildet ist. Mithin sind dann die zusammenwirkenden Lagerflächen ebenfalls eben oder im Wesentlichen eben ausgebildet.

Gemäß einer bevorzugten Ausführungsform der Erfindung kann vorgesehen sein, dass der Durchsteckschluss in Form eines einfachen Durchsteckschlusses ausgebildet ist und dass der zweite Verbindungsabschnitt nur einen zweiten Schlusssteg umfasst. Der einzige zweite Schlusssteg kann dann die erste Schlussdurchbrechung durchsetzen und wird dabei seitlich begrenzt durch die beiden ersten Schlussstege mit ihren aufeinander zuweisenden weiblichen Lagerflächen.

Ferner ist es günstig, wenn der Durchsteckschluss in Form eines doppelten Durchsteckschlusses ausgebildet ist, wenn der zweite Verbindungsabschnitt zwei zweite Schlussstege umfasst, welche eine zweite Schlussdurchbrechung seitlich begrenzen, wenn einer der zwei ersten Schlussstege die zweite Schlussdurchbrechung durchsetzt und wenn einer der zwei zweiten Schlussstege die erste Schlussdurchbrechung durchsetzt. Doppelte Durchsteckschlüsse haben insbesondere bei medizinischen Clips mit besonders langen Klemmarmen beziehungsweise Maulteilen den Vorteil, dass sie ein unerwünschtes "Scissoring", also das aneinander Abgleiten von seitlichen Kanten der Klemmarme beim Schließen des Clips, weitestgehend oder sogar vollständig vermeiden helfen. Das "Scissoring" birgt die Gefahr, dass Weichgewebe in unerwünschter Weise verletzt, schlimmstenfalls durchtrennt, werden kann. Dies soll nach Möglichkeit vermieden werden.

Vorzugsweise sind der erste Klemmarm ausgehend vom ersten Klemmarmende und der zweite Klemmarm ausgehend vom zweiten Klemmarmende in Richtung auf freie Enden derselben geradlinig oder gekrümmt oder abgewinkelt ausgebildet. So lassen sich medizinische Clips mit nahezu beliebig geformten Maulteilen ausbilden, um Aneurysmen unterschiedlichster Formen und Größen in menschlichen oder tierischen Patienten optimal behandeln zu können.

Vorzugsweise ist der Clip aus einem metallischen Werkstoff ausgebildet. Insbesondere kann so eine Stabilität des Clips in erforderlicher Weise sichergestellt werden. Günstig ist es, wenn der metallische Werkstoff Titan oder eine Titan enthaltende Legierung ist, beispielsweise Ti6AI4V. Mithin ist es also vorteilhaft, wenn der Clip aus einem körperverträglichen Werkstoff ausgebildet ist, um Abstoßungsreaktionen zu vermeiden.

Insbesondere dann, wenn die Lagerflächen aus Titan oder einer Titan enthaltenden Legierung ausgebildet sind, führt eine große Kontaktüberlappungsfläche zwischen den beiden zusammenwirkenden Lagerflächen zu einer unerwünscht hohen Reibung. Daher ist eine Verringerung mindestens einer Lagerfläche eines der mindestens zwei Lagerflächenpaare im Vergleich zur projizierten Schlussstegprojektionsfläche wir erläutert vorteilhaft.

Vorzugsweise sind die zusammenwirkenden Lagerflächen aus einem metallischen Werkstoff ausgebildet. Insbesondere kann es sich dabei um Titan oder eine Titan enthaltende Legierung handeln. Mithin ist es also vorteilhaft, wenn die zusammenwirkenden Lagerflächen aus einem körperverträglichen Werkstoff gebildet sind.

Gemäß einer weiteren bevorzugten Ausführungsform der Erfindung kann vorgesehen sein, dass das vorspannende Element in Form einer Schraubenfeder mit mindestens einer Windung ausgebildet ist. Insbesondere kann die Schraubenfeder mit mindestens etwa 1,5 Windungen ausgebildet sein. Beispielsweise kann das vorspannende Element aus einem durch Pressformen ausgebildeten Rohling durch Wickeln hergestellt werden.

Die Herstellung des medizinischen Clips lässt sich weiter vereinfachen, insbesondere die Ausbildung ebener Lagerflächen, wenn die ersten und/oder zweiten Schlussstege ohne Berücksichtigung der mindestens einen Vertiefung einen rechteckigen Querschnitt aufweisen.

Ferner kann es vorteilhaft sein, wenn mindestens eine Lagerfläche, insbesondere nur eine Lagerfläche oder beide Lagerflächen, der zwei zusammenwirkenden Lagerflächen eines Lagerflächenpaars mit einer Beschichtung versehen ist. Insbesondere kann die Beschichtung in Form einer korrosionsverringernden Beschichtung oder einer passivierenden Beschichtung ausgebildet sein.

Insbesondere günstig ist es, wenn die Beschichtung reibungsmindernd ausgebildet ist. So kann zusätzlich zur vorgeschlagenen Verringerung der Größe Lagerfläche durch Vorsehen mindestens einer Vertiefung die unerwünscht auftretende Reibung zwischen zusammenwirkenden Lagerflächen eines Lagerflächenpaars durch die reibungsmindernde Beschichtung weiter verringert werden.

Vorzugsweise ist die Beschichtung in Form einer Oxidschicht ausgebildet. Beispielsweise kann es sich dabei um Titanoxid handeln, wenn der medizinische Clip aus einem Titan enthaltenden Werkstoff ausgebildet ist. Eine solche Oxidschicht ist, insbesondere bei Titan, wesentlich härter als das Grundmaterial des Clips und neigt daher deutlich weniger zum Fressen, wenn die zusammenwirkenden Lagerflächen aneinander abgleiten.

Die Oxidschicht lässt sich auf einfache Weise galvanisch Aufbringen.

Vorzugsweise ist der medizinische Clip in Form eines Aneurysmenclips ausgebildet. So kann er insbesondere eingesetzt werden, um Aneurysmen, also insbesondere Aussackungen an Hohlorganen, zu behandeln.

Die vorstehende Beschreibung umfasst somit insbesondere die nachfolgend in Form durchnummerierter Sätze definierten Ausführungsformen medizinischer Clips:
1. Medizinischer Clip (10), insbesondere in Form eines Aneurysmenclips, welcher einen ersten Klemmarm (14), einen zweiten Klemmarm (16) und ein vorspannendes Element (12) mit einem ersten und einem zweiten Ende (18, 20) umfasst, wobei der erste Klemmarm (14) ein erstes Klemmarmende (24) aufweist, welches über einen ersten Verbindungsabschnitt (26) mit dem ersten Ende (18) des vorspannenden Elements (12) verbunden ist, wobei der zweite Klemmarm (16) ein zweites Klemmarmende (26) aufweist, welches über einen zweiten Verbindungsabschnitt (28) mit dem zweiten Ende (20) des vorspannenden Elements (12) verbunden ist, wobei der Clip (10) einen die zusammenwirkenden ersten und zweiten Verbindungsabschnitte (26, 28) umfassenden Durchsteckschluss (30) umfasst mit mindestens einer ersten, am ersten Verbindungsabschnitt (26) angeordneten oder ausgebildeten und von zwei ersten Schlussstegen (34, 36) seitlich begrenzten Schlussdurchbrechung (32) und mindestens einem vom zweiten Verbindungsabschnitt (28) umfassten, die erste Schlussdurchbrechung (32) durchsetzenden zweiten Schlusssteg (38), wobei die erste Schlussdurchbrechung (32) zwei aufeinander zu weisende weibliche Lagerflächen (42, 44; 48, 68) umfasst und wobei der mindestens eine zweite Schlusssteg (38) zwei voneinander weg und auf die weiblichen Lagerflächen (42, 44) hin weisende männliche Lagerflächen (46, 48; 44, 70) umfasst, wobei mindestens zwei Lagerflächenpaare (50, 52, 54) mit jeweils einer weiblichen und einer männlichen Lagerfläche (42, 46, 44, 48, 68, 70) gebildet sind und wobei jeweils die zusammenwirkenden weiblichen und männlichen Lagerflächen (42, 46, 44, 48, 68, 70) parallel zueinander verlaufen und eine Lagerebene (56, 58, 60) definieren, dadurch gekennzeichnet, dass sich eine durch senkrechte Projektion der ersten Schlussstege (34, 36) in die Lagerebene (56, 58, 60) definierte erste Schlussstegprojektionsfläche (94) und sich eine durch senkrechte Projektion des mindestens einen zweiten Schlussstegs (38, 40) in die Lagerebene (56, 58, 60) definierte zweite Schlussstegprojektionsfläche (96) überlappen und eine Projektionsüberlappungsfläche (98) definieren, dass mindestens eine der beiden zusammenwirkenden Lagerflächen (42, 46, 44, 48, 68, 70) mindestens eines, insbesondere jedes, der mindestens zwei Lagerflächenpaare (50, 52, 54) mindestens eine Vertiefung (72) aufweist derart, dass eine tatsächliche Größe der Lagerfläche (42, 46, 44, 48, 68, 70) parallel zur Lagerebene (56, 58, 60), welche Lagerfläche (42, 46, 44, 48, 68, 70) von dem die mindestens eine Vertiefung (72) umfassenden Schlusssteg (34, 36, 38, 40) definiert ist, kleiner ist als die vom Schlusssteg (34, 36, 38, 40), welcher die mindestens eine Vertiefung (72) umfasst, in die Lagerebene (56, 58, 60) projizierte Schlussstegprojektionsfläche (94, 96), dass die beiden zusammenwirkenden und flächig aneinander anlegbaren oder anliegenden, zusammenwirkenden Lagerflächen (42, 46, 44, 48, 68, 70) mindestens eines, insbesondere jedes, Lagerflächenpaars (50, 52, 54) eine Kontaktüberlappungsfläche (74) definieren und dass ein Verhältnis der Kontaktüberlappungsfläche (74) und der Projektionsüberlappungsfläche (98) in einem Bereich von etwa 1/25 bis etwa 1/3 liegt.
2. Medizinischer Clip nach Satz 1, dadurch gekennzeichnet, dass die mindestens eine Vertiefung (72) in Form einer Nut (76) in der Lagerfläche oder in Form einer Abschrägung (82, 84) an der Lagerfläche (42, 46, 44, 48, 68, 70) ausgebildet ist.
3. Medizinischer Clip nach Satz 2, dadurch gekennzeichnet, dass sich die Nut (76) oder die Abschrägung (82, 84) parallel oder im Wesentlichen parallel zu einer vom jeweiligen Schlusssteg (34, 36, 38, 40) definierten Längsrichtung (78, 80) erstreckt.
4. Medizinischer Clip nach Satz 2 oder 3, dadurch gekennzeichnet, dass die Abschrägung (82, 84) eine Schrägfläche (86, 88) definiert, welche mit der Lagerebene (56, 58, 60) einen stumpfen Schrägenwinkel (90) einschließt, wobei insbesondere der Schrägenwinkel (90) einen Wert in einem Bereich von etwa 140° bis etwa 175° aufweist, insbesondere in einem Bereich von etwa 165° bis etwa 172°.
5. Medizinischer Clip nach einem der Sätze 2 bis 4, dadurch gekennzeichnet, dass sich die Nut (76) quer, insbesondere senkrecht, zu einer vom jeweiligen Schlusssteg (34, 36, 38, 40) definierten Längsrichtung (78, 80) erstreckt.
6. Medizinischer Clip nach einem der voranstehenden Sätze, dadurch gekennzeichnet, dass die mindestens eine Vertiefung (72) in Form eines Hohlkugelausschnitts (100) ausgebildet ist, insbesondere eine Form einer halben Hohlkugel aufweist.
7. Medizinischer Clip nach einem der voranstehenden Sätze, dadurch gekennzeichnet, dass die mindestens eine Vertiefung (72) durch eine den Schlusssteg (34, 36, 38, 40) durchsetzende Schlussstegdurchbrechung ausgebildet ist.
8. Medizinischer Clip nach Satz 7, dadurch gekennzeichnet, dass die Schlussstegdurchbrechung einen kreisförmigen Querschnitt aufweist.
9. Medizinischer Clip nach einem der voranstehenden Sätze, dadurch gekennzeichnet, dass mindestens eine der beiden zusammenwirkenden Lagerflächen (42, 46, 44, 48, 68, 70) mindestens eines, insbesondere jedes, der mindestens zwei Lagerflächenpaare (50, 52, 54) eine Mehrzahl von Vertiefungen (72) aufweist.
10. Medizinischer Clip nach Satz 9, dadurch gekennzeichnet, dass die Mehrzahl von Vertiefungen (72) identisch ausgebildet ist.
11. Medizinischer Clip nach Satz 9, dadurch gekennzeichnet, dass sich mindestens ein Teil der Mehrzahl von Vertiefungen (72) unterscheidet, insbesondere in ihrer Form und/oder Größe.
12. Medizinischer Clip nach einem der voranstehenden Sätze, dadurch gekennzeichnet, dass beide zusammenwirkenden Lagerflächen (42, 46, 44, 48, 68, 70) jedes Lagerflächenpaars (50, 52, 54) mindestens eine Vertiefung (72) umfassen.
13. Medizinischer Clip nach einem der voranstehenden Sätze, dadurch gekennzeichnet, dass eine Breite (92) der Schlussstege (34, 36, 38, 40) ohne Berücksichtigung der mindestens einen Vertiefung (72) quer zu einer vom jeweiligen Schlusssteg (34, 36, 38, 40) definierten Längsrichtung (78, 80) in einem Bereich von etwa 0,9 mm bis etwa 2,1 mm liegt.
14. Medizinischer Clip nach einem der voranstehenden Sätze, dadurch gekennzeichnet, dass der erste Klemmarm (14) und der zweite Klemmarm (16) in einer Grundstellung des Clips (10) einander maximal angenähert sind, insbesondere aneinander anliegend, und entgegen der Wirkung des vorspannenden Elements (12) von der Grundstellung in eine Öffnungsstellung voneinander weg bewegbar sind.
15. Medizinischer Clip nach einem der voranstehenden Sätze, dadurch gekennzeichnet, dass die Lagerebene (56, 58, 60) eben oder im Wesentlichen eben ausgebildet ist.
16. Medizinischer Clip nach einem der voranstehenden Sätze, dadurch gekennzeichnet, dass der Durchsteckschluss (30) in Form eines einfachen Durchsteckschlusses (62) ausgebildet ist und dass der zweite Verbindungsabschnitt (28) nur einen zweiten Schlusssteg (38) umfasst.
17. Medizinischer Clip nach einem der Sätze 1 bis 15, dadurch gekennzeichnet, dass der Durchsteckschluss (30) in Form eines doppelten Durchsteckschlusses (64) ausgebildet ist, dass der zweite Verbindungsabschnitt (28) zwei zweite Schlussstege (38, 40) umfasst, welche eine zweite Schlussdurchbrechung (66) seitlich begrenzen, dass einer der zwei ersten Schlussstege (34, 36) die zweite Schlussdurchbrechung (66) durchsetzt und dass einer der zwei zweiten Schlussstege (38, 40) die erste Schlussdurchbrechung (32) durchsetzt.
18. Medizinischer Clip nach einem der voranstehenden Sätze, dadurch gekennzeichnet, dass der erste Klemmarm (14) ausgehend vom ersten Klemmarmende (22) und der zweite Klemmarm (16) ausgehend vom zweiten Klemmarmende (24) in Richtung auf freie Enden (106, 108) derselben geradlinig oder gekrümmt oder abgewinkelt ausgebildet sind.
19. Medizinischer Clip nach einem der voranstehenden Sätze, dadurch gekennzeichnet, dass der Clip (10) aus einem metallischen Werkstoff ausgebildet ist, insbesondere aus Titan oder einer Titan enthaltenden Legierung, insbesondere Ti6AI4V.
20. Medizinischer Clip nach einem der voranstehenden Sätze, dadurch gekennzeichnet, dass die zusammenwirkenden Lagerflächen (42, 46, 44, 48, 68, 70) aus einem metallischen Werkstoff, insbesondere aus Titan oder einer Titan enthaltenden Legierung, ausgebildet sind.
21. Medizinischer Clip nach einem der voranstehenden Sätze, dadurch gekennzeichnet, dass das vorspannende Element (12) in Form einer Schraubenfeder (110) mit mindestens einer Windung, insbesondere mit mindestens etwa 1,5 Windungen, ausgebildet ist.
22. Medizinischer Clip nach einem der voranstehenden Sätze, dadurch gekennzeichnet, dass die ersten und/oder zweiten Schlussstege (34, 36, 38, 40) ohne Berücksichtigung der mindestens einen Vertiefung (72) einen rechteckigen Querschnitt aufweisen.
23. Medizinischer Clip nach einem der voranstehenden Sätze, dadurch gekennzeichnet, dass mindestens eine Lagerfläche (42, 46, 44, 48, 68, 70), insbesondere nur eine Lagerfläche (42, 46, 44, 48, 68, 70) oder beide Lagerflächen (42, 46, 44, 48, 68, 70), der zwei zusammenwirkenden Lagerflächen (42, 46, 44, 48, 68, 70) eines Lagerflächenpaars (50, 52, 54) mit einer Beschichtung versehen ist.
24. Medizinischer Clip nach Satz 23, dadurch gekennzeichnet, dass die Beschichtung reibungsmindernd ausgebildet ist.
25. Medizinischer Clip nach Satz 23 oder 24, dadurch gekennzeichnet, dass die Beschichtung in Form einer Oxidschicht ausgebildet ist.
26. Medizinischer Clip nach Satz 25, dadurch gekennzeichnet, dass die Oxidschicht galvanisch aufgebracht ist.
27. Medizinischer Clip nach einem der voranstehenden Sätze, dadurch gekennzeichnet, dass der medizinische Clip (10) in Form eines Aneurysmenclips (112) ausgebildet ist.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Gesamtansicht eines ersten Ausführungsbeispiels eines medizinischen Clips mit einfachem Durchsteckschluss;
- Figur 2:: eine schematische perspektivische Gesamtansicht eines zweiten Ausführungsbeispiels eines medizinischen Clips mit doppeltem Durchsteckschluss;
- Figur 3:: eine schematische Teilansicht eines ersten Verbindungsabschnitts des in Figur 1 dargestellten medizinischen Clips;
- Figur 4:: eine schematische Teilansicht eines zweiten Verbindungsabschnitts des medizinischen Clips aus Figur 1;
- Figur 5:: eine schematische Schnittansicht des medizinischen Clips aus Figur 1 im Bereich des Durchsteckschlusses;
- Figur 6:: eine schematische Schnittansicht des medizinischen Clips aus Figur 2 im Bereich des Durchsteckschlusses;
- Figur 7:: eine schematische Schnittansicht ähnlich Figur 5 eines weiteren Ausführungsbeispiels eines medizinischen Clips;
- Figur 8:: eine schematische Schnittansicht ähnlich Figur 6 eines weiteren Ausführungsbeispiels eines medizinischen Clips:
- Figur 9:: eine schematische Teilansicht ähnlich Figur 3 eines ersten Verbindungsabschnitts eines weiteren Ausführungsbeispiels eines medizinischen Clips;
- Figur 10:: eine Schnittansicht längs Linie 10-10 in Figur 9;
- Figur 11:: eine schematische Teilansicht ähnlich Figur 4 eines zweiten Verbindungsabschnitts eines weiteren Ausführungsbeispiels eines medizinischen Clips;
- Figur 12:: eine schematische, teilweise durchbrochene Draufsicht auf den Schlussbereich eines Ausführungsbeispiel eines medizinischen Clips, welcher gebildet ist durch die ersten und zweiten, in den Figuren 9 und 11 dargestellten Verbindungsabschnitte;
- Figur 12A:: eine vergrößerte schematische Ansicht des Bereichs A in Figur 12 zur Darstellung überlappender Schlussstegprojektionsflächen, welche die Projektionsüberlappungsfläche, sowie der Kontaktüberlappungsfläche zweier zusammenwirkender Lagerflächen;
- Figur 13:: eine schematische Teilansicht ähnlich Figur 9 eines ersten Verbindungsabschnitts eines weiteren Ausführungsbeispiels eines medizinischen Clips;
- Figur 14:: eine Schnittansicht längs Linie 14-14 in Figur 13;
- Figur 15:: eine schematische Teilansicht ähnlich Figur 11 eines zweiten Verbindungsabschnitts eines weiteren Ausführungsbeispiels eines medizinischen Clips;
- Figur 16:: eine schematische Teilansicht ähnlich Figur 9 eines ersten Verbindungsabschnitts eines weiteren Ausführungsbeispiels eines medizinischen Clips;
- Figur 17:: eine Schnittansicht längs Linie 17-17 in Figur 16;
- Figur 18:: eine schematische Teilansicht ähnlich Figur 11 eines zweiten Verbindungsabschnitts eines weiteren Ausführungsbeispiels eines medizinischen Clips; und
- Figur 19:: eine schematische Schnittansicht ähnlich Figur 5 eines weiteren Ausführungsbeispiels eines medizinischen Clips.

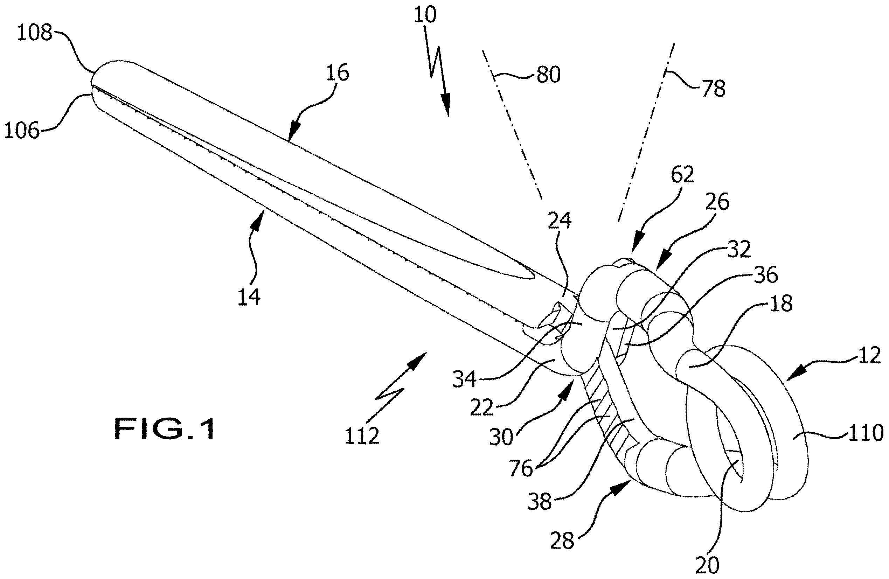

Ein erstes Ausführungsbeispiel eines medizinischen Clips ist schematisch in Figur 1 dargestellt und insgesamt mit dem Bezugszeichen 10 bezeichnet.

Der medizinische Clip 10 umfasst ein vorspannendes Element 12 und zwei Klemmarme, nämlich einen ersten Klemmarm 14 und einen zweiten Klemmarm 16.

Das vorspannende Element 12 weist ein erstes Ende 18 und ein zweites Ende 20 auf. Der erste Klemmarm 14 weist ein erstes Klemmarmende 22 auf. Der zweite Klemmarm 16 weist ein zweites Klemmarmende 24 auf.

Das erste Ende 18 des vorspannenden Elements 12 ist mit dem ersten Klemmarmende 22 über einen ersten Verbindungsabschnitt 26 verbunden. Das zweite Ende 20 des vorspannenden Elements 12 ist mit dem zweiten Klemmarmende 24 über einen zweiten Verbindungsabschnitt 28 verbunden.

Ein Schlussbereich des Clips 10 ist in Form eines Durchsteckschlusses 30 ausgebildet, welcher die beiden Verbindungsabschnitte 26 und 28 umfasst.

Der erste Verbindungsabschnitt 26 definiert eine Schlussdurchbrechung 32, welche von zwei ersten Schlussstegen 34 und 36 seitlich begrenzt ist. Der zweite Verbindungsabschnitt 28 umfasst einen einzigen, die erste Schlussdurchbrechung 32 durchsetzenden zweiten Schlusssteg 38.

Die erste Schlussdurchbrechung 32 umfasst zwei aufeinander zuweisende weibliche Lagerflächen 42 und 44. Der zweite Schlusssteg 38 umfasst zwei voneinander weg und auf die weiblichen Lagerflächen 42 und 44 hinweisende männliche Lagerflächen 46 und 48.

Der Durchsteckschluss 30 umfasst zwei Lagerflächenpaare 50 und 52. Das Lagerflächenpaar 50 umfasst die weibliche Lagerfläche 42 und die männliche Lagerfläche 46. Das Lagerflächenpaar 52 umfasst die weibliche Lagerfläche 44 und die männliche Lagerfläche 48.

Die zusammenwirkenden weiblichen und männlichen Lagerflächen 42, 46 beziehungsweise 44, 48 verlaufen jeweils parallel zueinander und definieren jeweils eine Lagerebene 56 beziehungsweise 58. Die Lagerebenen 56 und 58 verlaufen bei diesem Ausführungsbeispiel ebenfalls parallel zueinander.

Der Durchsteckschluss 30 ist in Form eines einfachen Durchsteckschlusses 62 ausgebildet. Der zweite Verbindungsabschnitt 28 umfasst nur einen einzigen, nämlich den zweiten Schlusssteg 38.

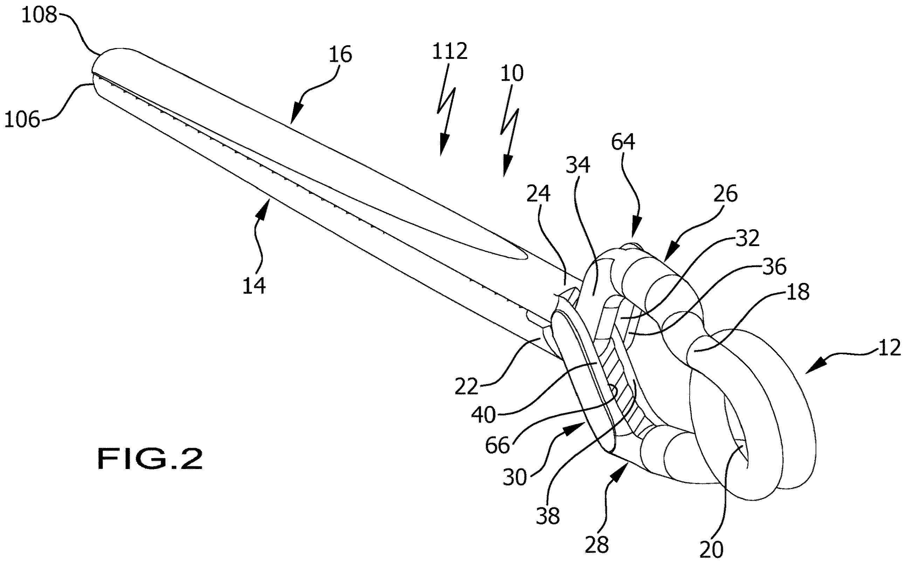

Ein zweites Ausführungsbeispiel eines medizinischen Clips 10 ist in Figur 2 schematisch dargestellt. Dieser Clip 10 stimmt in seinem Aufbau im Wesentlichen mit dem Ausführungsbeispiel des Clips 10 aus Figur 1 überein, sodass beim Ausführungsbeispiel der Figur 2 zur Bezeichnung identischer beziehungsweise funktional vergleichbarer Komponenten und Elemente dieselben Bezugszeichen verwendet werden.

Der medizinische Clip 10 aus Figur 2 unterscheidet sich vom ersten Ausführungsbeispiel des medizinischen Clips 10 aus Figur 1 durch die Ausgestaltung des Durchsteckschlusses 30. Beim Ausführungsbeispiel der Figur 2 ist der Durchsteckschluss 30 in Form eines doppelten Durchsteckschlusses 64 ausgebildet. Der zweite Verbindungsabschnitt 28 umfasst in diesem Fall zwei zweite Schlussstege 38 und 40. Der zweite Verbindungsabschnitt 28 umfasst ferner eine zweite Schlussdurchbrechung 66. Diese ist seitlich von den beiden zweiten Schlussstegen 38 und 40 begrenzt. Bei diesem doppeltem Durchsteckschluss 64 durchsetzt der Schlusssteg 38 analog wie beim Clip 10 der Figur 1 die erste Schlussdurchbrechung 32. Der erste Schlusssteg 34 durchsetzt im Fall des doppelten Durchsteckschlusses 64 jedoch die zweite Schlussdurchbrechung 66.

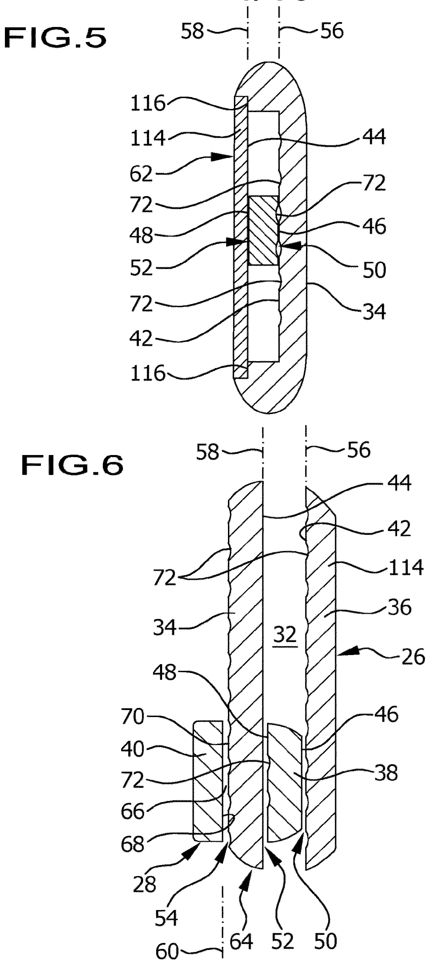

Wie in Figur 6 schematisch dargestellt ist am zweiten Schlusssteg 40 eine weitere weibliche Lagerfläche 68 ausgebildet, die mit einer männlichen Lagerfläche 70 am ersten Schlusssteg 34 zusammenwirkt. Die männliche Lagerfläche 48 bildet bei diesem doppelten Durchsteckschluss 64 also dem Grunde nach eine weibliche Lagerfläche der zweiten Schlussdurchbrechung 66. Die weibliche Lagerfläche 44 bildet in diesem Fall in analoger Weise in ihrer Funktion eine männliche Lagerfläche des ersten Schlussstegs 34 im Zusammenwirken mit der Lagerfläche 48.

Der doppelte Durchsteckschluss 64 umfasst drei Lagerflächenpaare 50, 52 und 54. Das Lagerflächenpaar 50 wird gebildet durch die zusammenwirkenden Lagerflächen 42 und 46. Das Lagerflächenpaar 52 wird gebildet durch die zusammenwirkenden Lagerflächen 44 und 48. Das Lagerflächenpaar 54 wird gebildet durch die zusammenwirkenden Lagerflächen 68 und 70.

Das eingangs erläuterte Problem bekannter medizinischer Clips, dass sie bei der Bestimmung der Schließkraft eine unzureichende Wiederholgenauigkeit aufweisen, wird bei nachfolgend in Verbindung mit den in den Figuren beschriebenen Ausführungsbeispielen medizinischer Clips 10 dadurch gelöst, dass mindestens eine der zusammenwirkenden Lagerflächen 42, 46, 44, 48 und gegebenenfalls 68, 70 mindestens eine Vertiefung aufweist, um die tatsächlich wirksame Größe der mit der mindestens einen Vertiefung 72 versehenen Lagerfläche im Zusammenwirken mit der entsprechend zugeordneten Lagerfläche zu verringern, sodass sich die Größe einer Kontaktüberlappungsfläche 74 ebenfalls verringert. Mindestvoraussetzung ist, dass mindestens eines der Lagerpaare 50, 52 und gegebenenfalls 54 bei einem Clip 10 mit doppeltem Durchsteckschluss 64 mindestens eine Lagerfläche mit mindestens einer Vertiefung aufweist. Die Kontaktüberlappungsfläche 74 wird definiert durch denjenigen Flächenbereich, in dem die beiden zusammenwirkenden und flächig aneinander anlegbaren oder anliegenden Lagerflächen 42 und 46 beziehungsweise 44 und 48 beziehungsweise 68 und 70 der Lagerflächenpaare 50, 52 beziehungsweise 54 in Kontakt stehen beziehungsweise beim Öffnen und Schlie-ßen des Clips 10 aneinander abgleiten.

Die mindestens eine Vertiefung 72 kann in unterschiedlicher Form ausgebildet sein, wie es nachfolgend in Verbindung mit den Figuren noch erläutert wird.

Wie definiert liegt die Kontaktüberlappungsfläche 74 in der jeweiligen Lagerebene 56, 58 beziehungsweise 60. Die Lagerebene 60 wird definiert durch die zusammenwirkenden Lagerflächen 68 und 70.

Vorab sei angemerkt, dass der Schlusssteg 38 beim einfachen Durchsteckschluss 62 sowie die Schlussstege 34 und 38 beim doppelten Durchsteckschluss 64 ohne Berücksichtigung der mindestens einen Vertiefung 72 einen rechteckigen Querschnitt aufweisen. Ein Querschnitt der Schlussstege 34 und 36 bei den Clips 10 mit einfachem Durchsteckschluss 62 ist im Wesentlichen langgestreckt oval. Die Querschnitte der Schlussstege 36 und 40 sind beim doppelten Durchsteckschluss 64 jeweils in etwa rechteckig.

Das Ausführungsbeispiel des Clips 10 gemäß den Figuren 1, 3, 4 und 5 weist eine Mehrzahl von Vertiefungen 72 auf. Die Vertiefungen 72 sind in Form von Nuten 76 in den Lagerflächen 42 und 46 ausgebildet, die beim Clip 10 zusammenwirken und das Lagerflächenpaar 50 definieren. Die Nuten 76 erstrecken sich bei dem Ausführungsbeispiel der Figuren 1, 3, 4 und 5 parallel zueinander und quer zu Längsrichtungen 78 beziehungsweise 80, die einerseits durch die ersten Schlussstege 34 und 36 und andererseits durch den Schlusssteg 38 definiert werden.

Die Nuten 76 sind bei dem dargestellten Ausführungsbeispiel gefräst, um so eine kleinere Oberfläche zu erreichen, mithin also eine wirksame Größe der Lagerflächen 42 und 46 zu verringern, um die Reibung zwischen diesen ebenfalls zu vermindern.

Die Nuten 76 weisen im Querschnitt eine Begrenzungslinie auf, die einen Kreisbogenausschnitt bildet. So ergibt sich im Übergang zu den Lagerflächen 42 und 46 ein stumpfer, nahezu gestreckter Winkel. Die Ausbildung von Graten wird auf diese Weise vermieden.

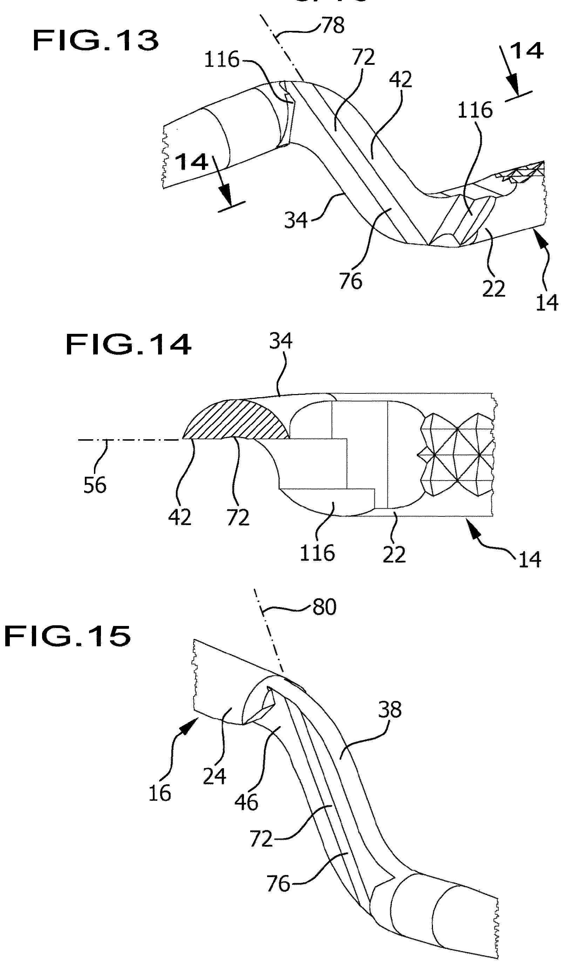

Bei dem schematisch in den Figuren 13 bis 15 teilweise dargestellten Ausführungsbeispiel eines medizinischen Clips 10 ist in den Lagerflächen 42 beziehungsweise 44 lediglich eine einzige Nut 76 ausgebildet. Diese erstreckt sich jeweils parallel oder im Wesentlichen parallel zu den von den Schlussstegen 34 beziehungsweise 38 definierten Längsrichtungen 78 beziehungsweise 80. Auch die Nuten bei diesem Ausführungsbeispiel sind im Querschnitt von einer Linie begrenzt, die einen Ausschnitt eines Kreisbogens definiert, sodass wiederum im Übergang zur verbleibenden, in ihrer Größe reduzierten Lagerfläche 42 beziehungsweise 46 ein stumpfer, nahezu gestreckter Winkel definiert wird. Die aneinander anliegenden Lagerflächen 42 und 46 sind somit in der gemeinsamen Lagerebene 56 auf den Bereich der Kontaktüberlappungsfläche 74 reduziert, wodurch die zwischen den Lagerflächen 42 und 46 auftretende Reibung im Vergleich zu Lagerflächen 42 und 46 ohne die Nut 76 vermindert ist.

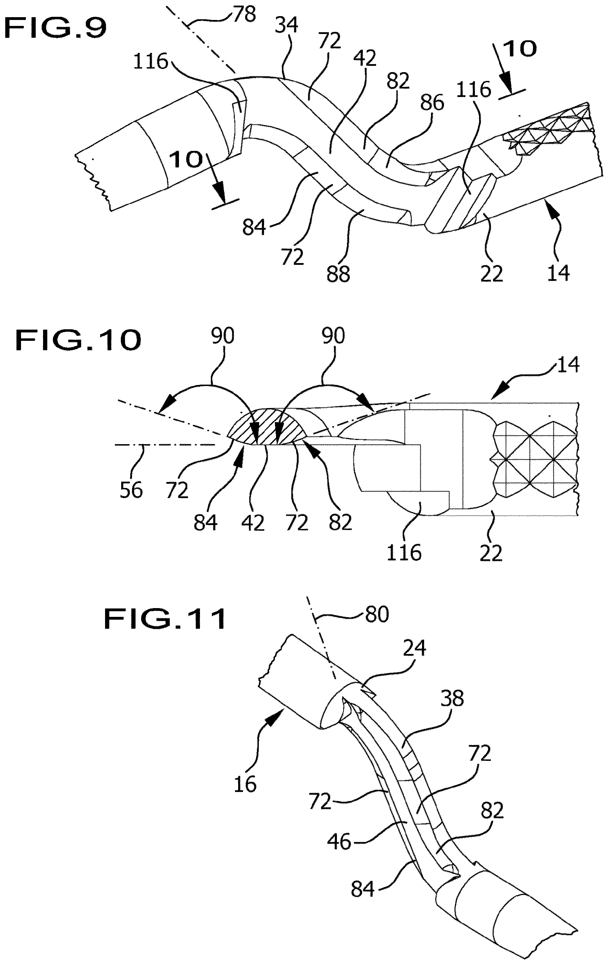

In einem weiteren Ausführungsbeispiel, das schematisch in den Figuren 9 bis 11 dargestellt ist, sind an den Schlussstegen 34 und 38 jeweils zwei Vertiefungen 72 in Form von Abschrägungen 82 und 84 ausgebildet. Diese Abschrägungen 82 und 84 sind an der Lagerfläche 42 beziehungsweise 46 ausgebildet und reduzieren eine für die gegenseitige Lagerung wirksame Größe derselben, die sich parallel zur Lagerebene 56 erstreckt. Somit verbleiben lediglich ein schmaler Streifen der Lagerfläche 42 parallel zur Lagerebene 56 und ein schmaler Streifen der Lagerfläche 46 parallel zur Lagerebene 56 am Schlusssteg 38. Die Abschrägungen 82 und 84 definieren Schrägflächen 86 und 88, welche mit der Lagerebene 56 einen stumpfen Schrägenwinkel 90 einschlie-ßen. Der Schrägenwinkel 90 weist einen Wert in einem Bereich von etwa 140° bis 175° auf. Bei dem in den Figuren dargestellten Ausführungsbeispiel liegt der Schrägenwinkel 90 in einem Bereich von etwa 165° bis 172°.

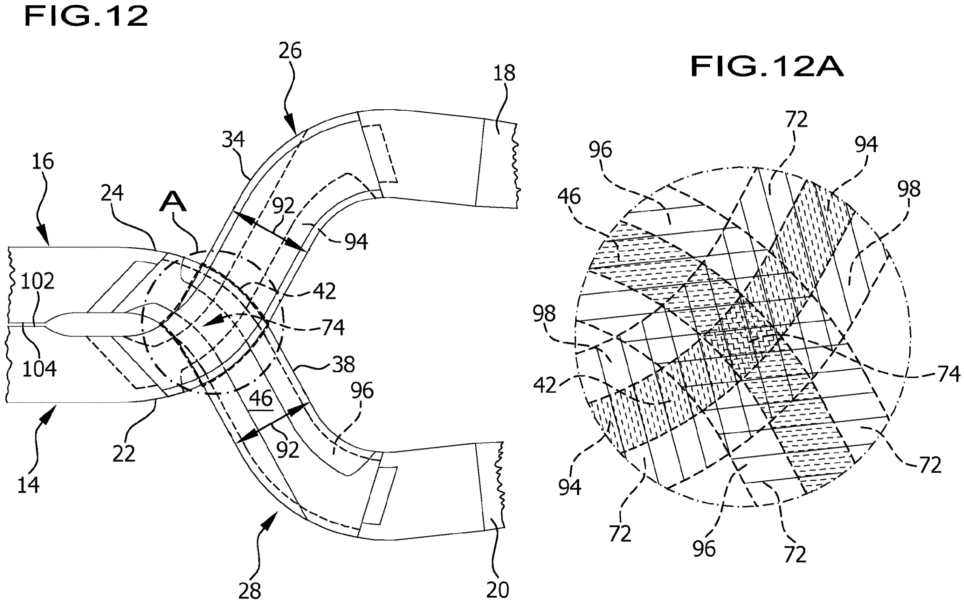

Durch die Ausbildung der Abschrägungen 82 und 86 verbleiben wie beschrieben von den Lagerflächen 42 und 46 nur schmale Streifen mit einer Streifenbreite, die etwa 1/3 einer Breite 92 der Schlussstege 34 und 38 entspricht. Die beiden Lagerflächen 42 und 46 können somit nur noch in einem Bereich, welcher durch die Kontaktüberlappungsfläche 74 definiert ist, flächig aneinander anliegen. Die Kontaktüberlappungsfläche 74 ist in den Figuren 12 und 12A als Überlappungsbereich der verbleibenden Lagerflächen 42 und 46 schematisch dargestellt. In Figur 12A ist sie durch doppelt gestrichelte Schraffur gekennzeichnet.

Durch eine senkrechte Projektion des ersten Schlussstegs 34 in die Lagerebene 56 wird eine erste Schlussstegprojektionsfläche 94 definiert. Eine senkrechte Projektion des zweiten Schlussstegs 38 in die Lagerebene 56 definiert eine zweite Schlussstegprojektionsfläche 96. Die Schlussstegprojektionsflächen 94 und 96 überlappen in der Lagerebene 56 und definieren so eine Projektionsüberlappungsfläche 98. Diese ist in den Figuren 12 und 12A schematisch dargestellt und in Figur 12A doppelt schraffiert dargestellt. Die Kontaktüberlappungsfläche 74, die einen Teil der Projektionsüberlappungsfläche 98 bildet, ist dagegen vierfach schraffiert dargestellt durch Überlagerung der doppelten Schraffur der Projektionsüberlappungsfläche 98 sowie der doppelt gestrichelten Schraffur.

Bei diesem Ausführungsbeispiel weisen die beiden Lagerflächen 42 und 46 des Lagerflächenpaars 50 mindestens eine Vertiefung 72, nämlich jeweils zwei Vertiefungen 72 in Form der Abschrägungen 82 und 84 auf, sodass eine tatsächliche Größe der Lagerflächen 42 und 46 parallel zur Lagerebene 56 kleiner ist als die von den Schlussstegen 34 und 38 in die Lagerebene 56 projizierten Schlussstegprojektionsflächen 94 und 96. Ein Verhältnis der Kontaktüberlappungsfläche 74 und der Projektionsüberlappungsfläche 98 liegt in einem Bereich von etwa 1/25 bis etwa 1/3 liegt. Bei dem Ausführungsbeispiel, wie es schematisch in den Figuren 9 bis 12 dargestellt ist, beträgt das Verhältnis etwa 1/9 unter der Annahme, dass durch die beiden Abschrägungen 82 und 84 jeweils etwa ein Drittel der Lagerflächen 42 und 46 parallel zur Lagerebene 56 entfernt sind. Die beiden verbleibenden Lagerflächen 42 und 46, die im Vergleich zu den ursprünglichen Lagerflächen 42 und 46, also ohne Berücksichtigung der Abschrägungen 82 und 84, mithin also ohne Berücksichtigung der beiden Vertiefungen 72, die Breite 92 aufweisen, wird somit auf ein Drittel bezogen auf die Breiten der ersten und zweiten Schlussstegprojektionsflächen 94 und 96 reduziert. Liegen die Lagerflächen 42 und 46 aneinander an, so ergibt sich das Produkt der verbleibenden, jeweils auf etwa 1/3 ihrer ursprünglich verringerten Lagerflächen 42 und 46 und somit eine Größe der Kontaktüberlappungsfläche 74, die nur noch etwa 1/9 der der Größe der Projektionsüberlappungsfläche 98 entspricht.

Die beschriebene Betrachtung der Verringerung der Größe der Kontaktüberlappungsfläche 74 unter Berücksichtigung der Ausbildung mindestens einer Vertiefung 72 in einer der beiden zusammenwirkenden Lagerflächen mindestens eines Lagerflächenpaars 50, 52 oder 54 ist entsprechend auch auf andere Formen von Vertiefungen 72 anwendbar. So lassen sich die Lagerflächen 42 und 46 auch bei quer zu den Längsrichtungen 78 und 80 ausgebildeten Nuten 76 in ähnlicher Weise reduzieren, wobei dann nicht nur eine einzige Kontaktüberlappungsfläche 74 definiert wird, sondern eine Vielzahl entsprechend kleinerer Kontaktüberlappungsflächen 74, die dann in der Summe wiederum einen Flächenwert aufweisen, welcher in einem Verhältnis in einem Bereich von etwa 1/25 bis etwa 1/3 bezogen auf die wie oben erläutert zu bestimmende Projektionsüberlappungsfläche 98 liegt. Auch für Nuten 76, die sich parallel zu den Längsrichtungen 78 und 80 erstrecken, ergibt sich eine analoge Betrachtungsweise. Hier ergeben sich dann vier Kontaktüberlappungsflächen 74 durch das in Kontakttreten der jeweils zwei, die Lagerflächen 42 und 46 verbleibenden, durch die Nut 76 getrennten Streifen, wobei eine Flächensumme der vier Kontaktüberlappungsflächen 74 dann im Verhältnis wiederum bezogen auf die Projektionsüberlappungsfläche 98 in einem Bereich von etwa 1/25 bis etwa 1/3 liegt.

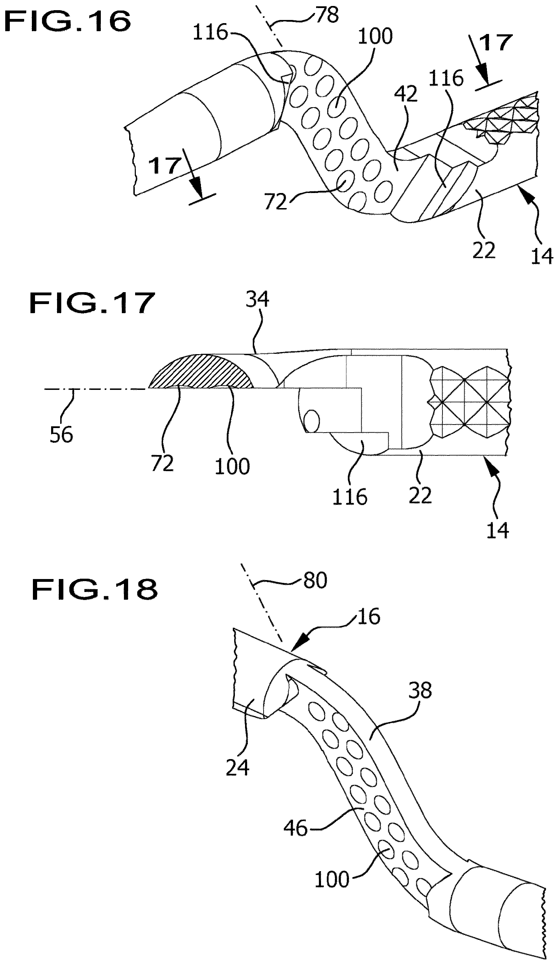

Die Figuren 16 bis 18 zeigen schematisch einen Ausschnitt der Verbindungsabschnitte 26 und 28 eines Clips 10. Bei diesem Ausführungsbeispiel ist eine Mehrzahl von Vertiefungen 72 in den Lagerflächen 42 und 46 ausgebildet, und zwar in Form von Hohlkugelausschnitten 100. Bei dem dargestellten Ausführungsbeispiel ist eine Tiefe der Vertiefung wesentlich kleiner als ein Radius der Hohlkugelausschnitte 100. Die Hohlkugelausschnitte 100 können insbesondere die Form einer halben Hohlkugel aufweisen. Auch hier sind die Vertiefungen 72 eingefräst, sodass sich ein stumpfer, nahezu gestreckter Winkel im Übergang zu den verbleibenden Lagerflächen 42 und 46 ergibt.

Auch bei diesem Ausführungsbeispiel sind die verbleibenden Lagerflächen 42 und 46 unter Berücksichtigung der Vertiefungen 72 signifikant reduziert im Vergleich zu den Lagerflächen 42 und 46 ohne Berücksichtigung der Vertiefungen 72. Ohne Berücksichtigung der Vertiefungen 72 ergeben sich auch hier wie oben erläutert erste und zweite Schlussstegprojektionsflächen 94 und 96 in der Lagerebene 56. Für die aneinander anliegenden Lagerflächen 42 und 46 mit den Vertiefungen 72 in Form von Hohlkugelausschnitten 100 verbleibt unter Berücksichtigung der Vertiefungen 72 lediglich eine Kontaktüberlappungsfläche 74 in einer Größe, dass sich auch hier ein Verhältnis der Kontaktüberlappungsfläche 74 und der Projektionsüberlappungsfläche 98 mit einem Wert in einem Bereich von etwa 1/25 bis etwa 1/3 ergibt.

Alternativ kann die mindestens eine Vertiefung 72 auch durch eine den jeweiligen Schlusssteg 34 beziehungsweise 38 durchsetzende Schlussstegdurchbrechung ausgebildet sein. Derartige Schlussstegdurchbrechungen ergeben eine Ansicht der Lagerflächen 42 und 46 analog zu den Figuren 16 und 18, und zwar dann, wenn die Schlussstegdurchbrechungen einen kreisförmigen Querschnitt aufweisen.

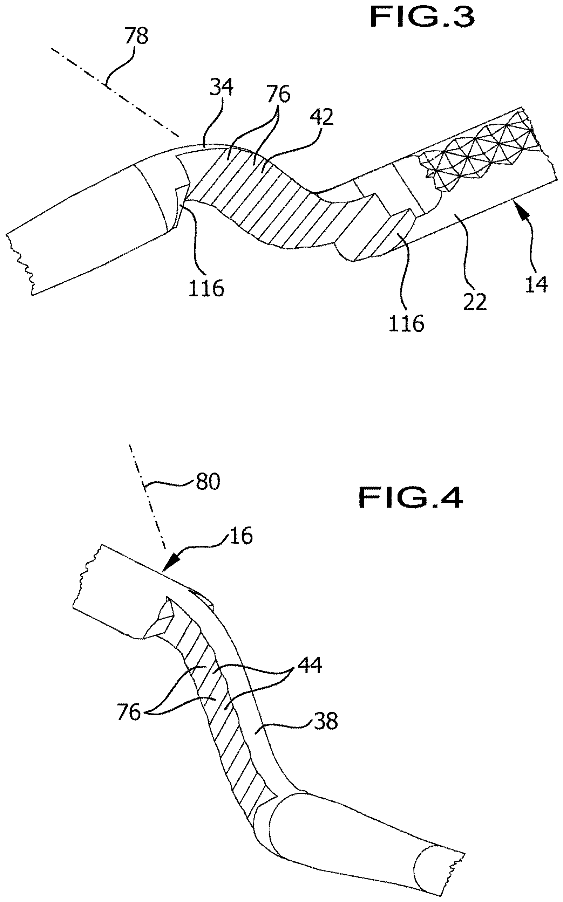

Bei den Ausführungsbeispielen der Figuren 3 und 4, 9 bis 11 sowie 16 bis 18 weist mindestens eine der beiden zusammenwirkenden Lagerflächen 42 und 46 des Lagerflächenpaars 50 eine Mehrzahl von Vertiefungen 72 auf.

Bei den Ausführungsbeispielen der Figuren 3 und 4 einerseits sowie 16 bis 18 ist die Mehrzahl von Vertiefungen 72 jeweils identisch ausgebildet.

Bei in den Figuren nicht dargestellten Ausführungsbeispielen ist mindestens ein Teil der Mehrzahl von Vertiefungen 72 unterschiedlich ausgebildet, und zwar wahlweise in ihrer Form und/oder ihrer Größe unterschiedlich.

Die bislang beschriebenen Ausführungsbeispiele sehen vor, dass die beiden zusammenwirkenden Lagerflächen 42 und 46 des Lagerflächenpaars 50 jeweils mindestens eine Vertiefung 72 umfassen.

Die Breite 92 der Schlussstege 34 und 38 weist, ohne Berücksichtigung der mindestens einen Vertiefung 72, quer zu den vom jeweiligen Längssteg 34 beziehungsweise 38 definierten Längsrichtung 78 beziehungsweise 80 einen Wert auf, welcher in einem Bereich von etwa 0,9 mm bis etwa 2,1 mm liegt. Beispielsweise können die Clips 10 insgesamt unterschiedliche Größen aufweisen, sodass die Breite 92, die letztlich durch einen Durchmesser eines Drahts vorgegeben wird, der das Ausgangsmaterial zur Herstellung der Clips 10 bildet, einen Wert aufweist, welcher beispielsweise etwa 1 mm, etwa 1,2 mm oder etwa 1,7 mm betragen kann.

Wie schematisch in den Figuren 1 und 2 dargestellt, sind die Klemmarme 14 und 16 in einer Grundstellung des jeweiligen Clips 10 einander maximal angenähert. Die Figuren 1 und 2 zeigen Ausführungsbeispiele von Clips 10, bei denen die Klemmarme 14 und 16 in der Grundstellung aneinander anliegen.

Die Klemmarme 14 und 16 können entgegen der Wirkung des vorspannenden Elements 12 aus der Grundstellung in eine Öffnungsstellung voneinander wegbewegt werden, sodass zwischen Klemmflächen 102 und 104 der Klemmarme 14 und 16 eine Aussackung eines Hohlorgans eingeführt und zwischen diesen geklemmt werden kann, und zwar indem der Clip 10 freigegeben wird, sodass das vorspannende Element 12 eine vorspannende Kraft ausüben kann, um die Klemmflächen 102 und 104 der Klemmarme 14 und 16 gegeneinander zu drücken.

Die Klemmarme 14 und 16 erstrecken sich vom ersten Klemmarmende 22 beziehungsweise vom zweiten Klemmarmende 24 in Richtung auf freie Enden 106, 108 hin.

Bei den Ausführungsbeispielen der Clips 10 der Figuren 1 und 2 verlaufen die Klemmarme 14 und 16 geradlinig. Bei alternativen, nicht dargestellten Ausführungsbeispielen verlaufen die Klemmarme 14 und 16 gekrümmt oder sind abgewinkelt, wobei jeweils sichergestellt sein muss, dass die Klemmflächen 102 und 104 in der Grundstellung im Wesentlichen über die gesamte Erstreckung von den Klemmarmenden 22 und 24 bis zu den freien Enden 106 und 108 flächig aneinander anliegen.

Das vorspannende Element 12 ist bei den in den Figuren 1 und 2 dargestellten Ausführungsbeispielen in Form einer Schraubenfeder 110 mit mindestens einer Windung ausgebildet. Die Ausführungsbeispiele der Figuren 1 und 2 umfassen Schraubenfedern 110 mit etwa 1,5 Windungen.

Der medizinische Clip 10 ist bei allen Ausführungsbeispielen in Form eines Aneurysmenclips 112 ausgebildet.

Der Clip 10 ist aus einem metallischen Werkstoff ausgebildet. Bei den in den Figuren dargestellten Ausführungsbeispielen besteht der Clip 10 aus Titan oder einer Titan enthaltenden Legierung, beispielsweise Ti6AI4V.

Ein aus den genannten metallischen Werkstoffen ausgebildeter Clip 10 weist Lagerflächen 42, 44, 46 und 48 sowie gegebenenfalls 68 und 70 auf, die dann aus dem metallischen Werkstoff, nämlich Titan oder einer Titan enthaltenden Legierung ausgebildet sind.

Mindestens eine der Lagerflächen 42, 44, 46 und 48 sowie gegebenenfalls 68 und 70, die die Lagerflächenpaare 50, 52 und gegebenenfalls 54 bilden, ist in nicht näher dargestellter Weise mit einer Beschichtung versehen. Insbesondere ist jeweils mindestens eine Lagerfläche jedes Lagerflächenpaars 50, 52 und gegebenenfalls 54 mit einer Beschichtung versehen. Eine solche Beschichtung ist eine optionale Ausgestaltung der Clips 10. Beispielsweise kann nur eine der Lagerflächen 42 und 46 oder es können auch beide Lagerflächen 42 und 46 mit einer Beschichtung versehen sein. Dies gilt entsprechend auch für weitere Lagerflächenpaare 52 und gegebenenfalls 54.

Die Beschichtung ist vorzugsweise reibungsmindernd ausgebildet.

Bei metallischen Werkstoffen, die zur Herstellung des Clips 10 eingesetzt werden, ist die Beschichtung vorzugsweise in Form einer Oxidschicht ausgebildet. Bei Clips 10 aus Titan oder einer Titan enthaltenden Legierung handelt es sich bei der Oxidschicht vorzugsweise um eine Titanoxid-Schicht.

Die Oxidschicht zu Beschichtung der Lagerflächen 42, 44, 46 und 48 sowie gegebenenfalls 68 und 70 ist vorzugsweise galvanisch aufgebracht.

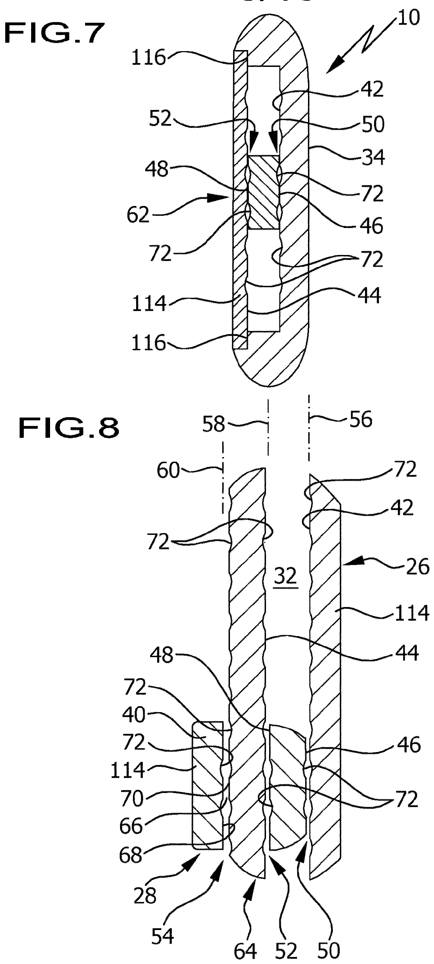

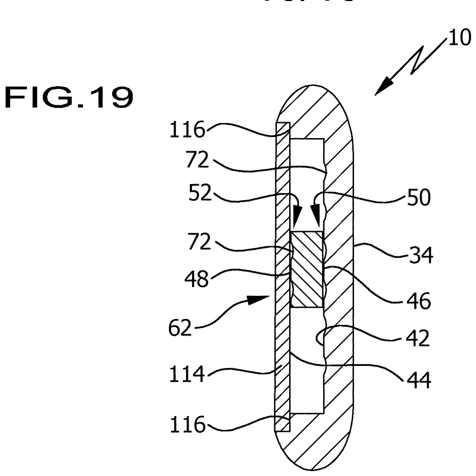

Die Figuren 7 und 19 zeigen schematisch weitere Ausführungsbeispiele eines Clips 10 mit einfachem Durchsteckschluss 62. Beim Ausführungsbeispiel der Figur 7 sind wie beim Ausführungsbeispiel der Figur 5 sowohl die Lagerflächen 42 und 46, die zusammen das Lagerflächenpaar 50 bilden, mit Vertiefungen 72 versehen als auch die Lagerflächen 44 und 48, die zusammen das Lagerflächenpaar 52 definieren. Beim Ausführungsbeispiel der Figur 5 sind die zusammenwirkenden Lagerflächen 44 und 48 jeweils ohne Vertiefungen 72 ausgebildet. Beim Ausführungsbeispiel der Figur 19 sind lediglich in den Lagerflächen 42 und 48 Vertiefungen ausgebildet. Die Lagerflächen 44 und 46 sind ohne Vertiefungen 72 ausgebildet, mithin also vertiefungsfrei. Somit weist beim Ausführungsbeispiel der Figur 19 jedes Lagerflächenpaar 50 beziehungsweise 52 lediglich eine Lagerfläche 42 beziehungsweise 48 auf, die mit mindestens einer Vertiefung 72 versehen ist.

Den Ausführungsbeispielen der Figuren 5, 7 und 19 ist gemein, dass mindestens eines der Lagerflächenpaare 50 und 52 mindestens eine Lagerfläche 42 und 46 beziehungsweise 42, 46, 44 und 48 beziehungsweise 42 und 48 aufweist, die mit mindestens einer Vertiefung 72 versehen ist.

Der zweite Schlusssteg 36 des ersten Verbindungsabschnitts 26 ist bei den Ausführungsbeispielen der Figuren 5, 7 und 19 durch ein sogenanntes Schlussplättchen 114 ausgebildet. Es ist in zwei Rücksprünge 116 am ersten Verbindungsabschnitt 26 eingesetzt und mit diesem verschweißt. Es wird eingesetzt und verschweißt, nachdem der jeweilige Clip 10 aus einem drahtförmigen Rohling durch entsprechendes Umformen ausgebildet ist.

Die Figuren 6 und 8 zeigen schematisch Varianten unterschiedlicher Clips 10 mit doppelten Durchsteckschlüssen 64. Beim Ausführungsbeispiel der Figur 6 sind die Lagerflächen 46, 44 und 68 ohne Vertiefungen 72 ausgebildet, mithin also vertiefungsfrei. Vertiefungen 72 sind dagegen in den Lagerflächen 42, 48 und 70 ausgebildet. Somit ist mindestens eine der beiden Lagerflächen jedes Lagerflächenpaars 50, 52 und 54 mit mindestens einer Vertiefung 72 versehen.

Beim schematisch in Figur 8 dargestellten Ausführungsbeispiel sind alle sechs Lagerflächen 42, 44, 46, 48, 68 und 70 mit mindestens einer Vertiefung 72 versehen. Somit sind auch bei diesem Ausführungsbeispiel die zusammenwirkenden und jeweils die Lagerflächenpaare 50, 52 und 54 ausbildenden Lagerflächen 42, 44, 46, 48, 68 und 70 mit mindestens einer Vertiefung 72 versehen.

Bei den Ausführungsbeispielen der Figuren 2, 6 und 8 sind der erste Schlusssteg 36 sowie der zweite Schlusssteg 40 in Form von Schlussplättchen 114 ausgebildet. Diese sind in nicht näher dargestellter Weise analog wie oben in Verbindung mit den Figuren 5, 7 und 19 bei einem einfachen Durchsteckschluss 62 erläutert, in dafür vorgesehen Rücksprünge an den beiden Verbindungsabschnitten 26 und 28 eingesetzt und verschweißt.

Die oben beschriebenen Ausführungsbeispiele medizinischer Clips 10 dienen lediglich der Erläuterung und sind daher nicht abschließen. So können insbesondere Anzahl, Form und Größe der dargestellten und beschriebenen Vertiefungen 72 beliebig miteinander kombiniert und gegebenenfalls variiert werden.

Beispielsweise können auf einer Lagerfläche eine Nut 76 und auf der mit dieser zusammenwirkenden Lagerfläche eine oder zwei Abschrägungen 82, 84 oder ein oder mehrere Hohlkugelausschnitte 100 ausgebildet sein. Es können Nuten 76 einerseits parallel zu einer Längsrichtung 78, 80 und andererseits quer dazu miteinander kombiniert werden zur Ausbildung eines Lagerflächenpaars 50, 52 oder 54.

Insbesondere bei den schematisch dargestellten Schnittansichten der Figuren 5 bis 8 und 19 dienen die schematisch eingezeichneten Vertiefungen 72 lediglich als Platzhalter. Es können hier wiederum Nuten, Abschrägungen und/oder Hohlkugelausschnitte sowie auch andere Formen zur Ausbildung der Vertiefungen 72 vorgesehen sein.

Bei den beschriebenen Ausführungsbeispielen medizinischer Clips 10 wird insbesondere die gewünschte Reibungsminderung dadurch erreicht, dass bei ihnen ein Verhältnis der Kontaktüberlappungsfläche 74 und der Projektionsüberlappungsfläche 90 in einem Bereich von etwa 1/25 bis etwa 1/3 liegt, vorzugsweise in einem Bereich von etwa 1/25 bis etwa 1/4. Je nach Wahl und Ausgestaltung der Vertiefungen 72 werden diese in Form und Größe so gewählt, dass die jeweiligen Lagerflächen, die miteinander zusammenwirken, so groß sind, dass der angegebene Verhältnisbereich erfüllt ist.

Durch die Reduzierung der Kontaktüberlappungsfläche 74 bezogen auf die Projektionsüberlappungsfläche 98 durch Vorsehen mindestens einer Vertiefung 72 in mindestens einer Lagerfläche 42, 44, 46 48, 68 und 70 eines Lagerflächenpaars 50, 52 oder 54 des Clips 10 lässt sich ein Übergang von Haftreibung auf Gleitreibung bei mindestens einem Lagerflächenpaar 50, 52 und 54 verringern. Dadurch wird dann wie eingangs erläutert eine Wiederholgenauigkeit bei der Bestimmung der Schließkraft des Clips 10 erhöht beziehungsweise eine Wiederholungenauigkeit in der gewünschten Weise verringert.

### Bezugszeichenliste

- 10: Clip
- 12: vorspannendes Element
- 14: erster Klemmarm
- 16: zweiter Klemmarm
- 18: erstes Ende
- 20: zweites Ende
- 22: erstes Klemmarmende
- 24: zweites Klemmarmende
- 26: erster Verbindungsabschnitt
- 28: zweiter Verbindungsabschnitt
- 30: Durchsteckschluss
- 32: erste Schlussdurchbrechung
- 34: erster Schlusssteg
- 36: erster Schlusssteg
- 38: zweiter Schlusssteg
- 40: zweiter Schlusssteg
- 42: weibliche Lagerfläche
- 44: weibliche Lagerfläche
- 46: männliche Lagerfläche
- 48: männliche Lagerfläche
- 50: Lagerflächenpaar
- 52: Lagerflächenpaar
- 54: Lagerflächenpaar
- 56: Lagerebene
- 58: Lagerebene
- 60: Lagerebene
- 62: einfacher Durchsteckschluss
- 64: doppelter Durchsteckschluss
- 66: zweite Schlussdurchbrechung
- 68: weibliche Lagerfläche
- 70: männliche Lagerfläche
- 72: Vertiefung
- 74: Kontaktüberlappungsfläche
- 76: Nut
- 78: Längsrichtung
- 80: Längsrichtung
- 82: Abschrägung
- 84: Abschrägung
- 86: Schrägfläche
- 88: Schrägfläche
- 90: Schrägenwinkel
- 92: Breite
- 94: erste Schlussstegprojektionsfläche
- 96: zweite Schlussstegprojektionsfläche
- 98: Projektionsüberlappungsfläche
- 100: Hohlkugelausschnitt
- 102: Klemmfläche
- 104: Klemmfläche
- 106: freies Ende
- 108: freies Ende
- 110: Schraubenfeder
- 112: Aneurysmenclip
- 114: Schlussplättchen

## Patentansprüche

1. Medizinischer Clip (10), insbesondere in Form eines Aneurysmenclips, welcher einen ersten Klemmarm (14), einen zweiten Klemmarm (16) und ein vorspannendes Element (12) mit einem ersten und einem zweiten Ende (18, 20) umfasst, wobei der erste Klemmarm (14) ein erstes Klemmarmende (24) aufweist, welches über einen ersten Verbindungsabschnitt (26) mit dem ersten Ende (18) des vorspannenden Elements (12) verbunden ist, wobei der zweite Klemmarm (16) ein zweites Klemmarmende (26) aufweist, welches über einen zweiten Verbindungsabschnitt (28) mit dem zweiten Ende (20) des vorspannenden Elements (12) verbunden ist, wobei der Clip (10) einen die zusammenwirkenden ersten und zweiten Verbindungsabschnitte (26, 28) umfassenden Durchsteckschluss (30) umfasst mit mindestens einer ersten, am ersten Verbindungsabschnitt (26) angeordneten oder ausgebildeten und von zwei ersten Schlussstegen (34, 36) seitlich begrenzten Schlussdurchbrechung (32) und mindestens einem vom zweiten Verbindungsabschnitt (28) umfassten, die erste Schlussdurchbrechung (32) durchsetzenden zweiten Schlusssteg (38), wobei die erste Schlussdurchbrechung (32) zwei aufeinander zu weisende weibliche Lagerflächen (42, 44; 48, 68) umfasst und wobei der mindestens eine zweite Schlusssteg (38) zwei voneinander weg und auf die weiblichen Lagerflächen (42, 44) hin weisende männliche Lagerflächen (46, 48; 44, 70) umfasst, wobei mindestens zwei Lagerflächenpaare (50, 52, 54) mit jeweils einer weiblichen und einer männlichen Lagerfläche (42, 46, 44, 48, 68, 70) gebildet sind und wobei jeweils die zusammenwirkenden weiblichen und männlichen Lagerflächen (42, 46, 44, 48, 68, 70) parallel zueinander verlaufen und eine Lagerebene (56, 58, 60) definieren, **dadurch gekennzeichnet, dass** sich eine durch senkrechte Projektion der ersten Schlussstege (34, 36) in die Lagerebene (56, 58, 60) definierte erste Schlussstegprojektionsfläche (94) und sich eine durch senkrechte Projektion des mindestens einen zweiten Schlussstegs (38, 40) in die Lagerebene (56, 58, 60) definierte zweite Schlussstegprojektionsfläche (96) überlappen und eine Projektionsüberlappungsfläche (98) definieren, dass mindestens eine der beiden zusammenwirkenden Lagerflächen (42, 46, 44, 48, 68, 70) mindestens eines, insbesondere jedes, der mindestens zwei Lagerflächenpaare (50, 52, 54) mindestens eine Vertiefung (72) aufweist derart, dass eine tatsächliche Größe der Lagerfläche (42, 46, 44, 48, 68, 70) parallel zur Lagerebene (56, 58, 60), welche Lagerfläche (42, 46, 44, 48, 68, 70) von dem die mindestens eine Vertiefung (72) umfassenden Schlusssteg (34, 36, 38, 40) definiert ist, kleiner ist als die vom Schlusssteg (34, 36, 38, 40), welcher die mindestens eine Vertiefung (72) umfasst, in die Lagerebene (56, 58, 60) projizierte Schlussstegprojektionsfläche (94, 96), dass die beiden zusammenwirkenden und flächig aneinander anlegbaren oder anliegenden, zusammenwirkenden Lagerflächen (42, 46, 44, 48, 68, 70) mindestens eines, insbesondere jedes, Lagerflächenpaars (50, 52, 54) eine Kontaktüberlappungsfläche (74) definieren und dass ein Verhältnis der Kontaktüberlappungsfläche (74) und der Projektionsüberlappungsfläche (98) in einem Bereich von etwa 1/25 bis etwa 1/3 liegt.

2. Medizinischer Clip nach Anspruch 1, **dadurch gekennzeichnet, dass** die mindestens eine Vertiefung (72) in Form einer Nut (76) in der Lagerfläche oder in Form einer Abschrägung (82, 84) an der Lagerfläche (42, 46, 44, 48, 68, 70) ausgebildet ist.

3. Medizinischer Clip nach Anspruch 2, **dadurch gekennzeichnet, dass** sich die Nut (76) oder die Abschrägung (82, 84) parallel oder im Wesentlichen parallel zu einer vom jeweiligen Schlusssteg (34, 36, 38, 40) definierten Längsrichtung (78, 80) erstreckt.

4. Medizinischer Clip nach Anspruch 2 oder 3, **dadurch gekennzeichnet, dass** die Abschrägung (82, 84) eine Schrägfläche (86, 88) definiert, welche mit der Lagerebene (56, 58, 60) einen stumpfen Schrägenwinkel (90) einschließt, wobei insbesondere der Schrägenwinkel (90) einen Wert in einem Bereich von etwa 140° bis etwa 175° aufweist, insbesondere in einem Bereich von etwa 165° bis etwa 172°.

5. Medizinischer Clip nach einem der Ansprüche 2 bis 4, **dadurch gekennzeichnet, dass** sich die Nut (76) quer, insbesondere senkrecht, zu einer vom jeweiligen Schlusssteg (34, 36, 38, 40) definierten Längsrichtung (78, 80) erstreckt.

6. Medizinischer Clip nach einem der voranstehenden Ansprüche, **dadurch gekennzeichnet, dass** die mindestens eine Vertiefung (72) in Form eines Hohlkugelausschnitts (100) ausgebildet ist, insbesondere eine Form einer halben Hohlkugel aufweist.

7. Medizinischer Clip nach einem der voranstehenden Ansprüche, **dadurch gekennzeichnet, dass** die mindestens eine Vertiefung (72) durch eine den Schlusssteg (34, 36, 38, 40) durchsetzende Schlussstegdurchbrechung ausgebildet ist,
wobei insbesondere die Schlussstegdurchbrechung einen kreisförmigen Querschnitt aufweist.

8. Medizinischer Clip nach einem der voranstehenden Ansprüche, **dadurch gekennzeichnet, dass** mindestens eine der beiden zusammenwirkenden Lagerflächen (42, 46, 44, 48, 68, 70) mindestens eines, insbesondere jedes, der mindestens zwei Lagerflächenpaare (50, 52, 54) eine Mehrzahl von Vertiefungen (72) aufweist,
wobei insbesondere
a) die Mehrzahl von Vertiefungen (72) identisch ausgebildet ist
oder
b) sich mindestens ein Teil der Mehrzahl von Vertiefungen (72) unterscheidet, insbesondere in ihrer Form und/oder Größe.

9. Medizinischer Clip nach einem der voranstehenden Ansprüche, **dadurch gekennzeichnet, dass** beide zusammenwirkenden Lagerflächen (42, 46, 44, 48, 68, 70) jedes Lagerflächenpaars (50, 52, 54) mindestens eine Vertiefung (72) umfassen.

10. Medizinischer Clip nach einem der voranstehenden Ansprüche, **dadurch gekennzeichnet, dass** eine Breite (92) der Schlussstege (34, 36, 38, 40) ohne Berücksichtigung der mindestens einen Vertiefung (72) quer zu einer vom jeweiligen Schlusssteg (34, 36, 38, 40) definierten Längsrichtung (78, 80) in einem Bereich von etwa 0,9 mm bis etwa 2,1 mm liegt.

11. Medizinischer Clip nach einem der voranstehenden Ansprüche, **dadurch gekennzeichnet, dass** der Durchsteckschluss (30) in Form eines
a) einfachen Durchsteckschlusses (62) ausgebildet ist und dass der zweite Verbindungsabschnitt (28) nur einen zweiten Schlusssteg (38) umfasst
oder
b) doppelten Durchsteckschlusses (64) ausgebildet ist, dass der zweite Verbindungsabschnitt (28) zwei zweite Schlussstege (38, 40) umfasst, welche eine zweite Schlussdurchbrechung (66) seitlich begrenzen, dass einer der zwei ersten Schlussstege (34, 36) die zweite Schlussdurchbrechung (66) durchsetzt und dass einer der zwei zweiten Schlussstege (38, 40) die erste Schlussdurchbrechung (32) durchsetzt.

12. Medizinischer Clip nach einem der voranstehenden Ansprüche, **dadurch gekennzeichnet, dass** der erste Klemmarm (14) ausgehend vom ersten Klemmarmende (22) und der zweite Klemmarm (16) ausgehend vom zweiten Klemmarmende (24) in Richtung auf freie Enden (106, 108) derselben geradlinig oder gekrümmt oder abgewinkelt ausgebildet sind.

13. Medizinischer Clip nach einem der voranstehenden Ansprüche, **dadurch gekennzeichnet, dass**
a) der Clip (10) aus einem metallischen Werkstoff ausgebildet ist, insbesondere aus Titan oder einer Titan enthaltenden Legierung, insbesondere Ti6Al4V,
und/oder
b) die zusammenwirkenden Lagerflächen (42, 46, 44, 48, 68, 70) aus einem metallischen Werkstoff, insbesondere aus Titan oder einer Titan enthaltenden Legierung, ausgebildet sind.

14. Medizinischer Clip nach einem der voranstehenden Ansprüche, **dadurch gekennzeichnet, dass** die ersten und/oder zweiten Schlussstege (34, 36, 38, 40) ohne Berücksichtigung der mindestens einen Vertiefung (72) einen rechteckigen Querschnitt aufweisen.

15. Medizinischer Clip nach einem der voranstehenden Ansprüche, **dadurch gekennzeichnet, dass** mindestens eine Lagerfläche (42, 46, 44, 48, 68, 70), insbesondere nur eine Lagerfläche (42, 46, 44, 48, 68, 70) oder beide Lagerflächen (42, 46, 44, 48, 68, 70), der zwei zusammenwirkenden Lagerflächen (42, 46, 44, 48, 68, 70) eines Lagerflächenpaars (50, 52, 54) mit einer Beschichtung versehen ist,
wobei insbesondere
a) die Beschichtung reibungsmindernd ausgebildet ist
und/oder
b) die Beschichtung in Form einer Oxidschicht ausgebildet ist, wobei insbesondere die Oxidschicht galvanisch aufgebracht ist.
